# EUROPEAN PATENT SPECIFICATION

(11) **EP 3 166 780 B1**
(45) Date of publication and mention of the grant of the patent: **20.02.2019**
(21) Application number: 15818300.4
(22) Date of filing: 06.07.2015
(51) Int. Cl.: B32B 3/02, A44B 18/00

(54) **SURFACE FASTENER LOOP MEMBER AND SANITARY ARTICLE**
OBERFLÄCHENHAFTVERSCHLUSSLASCHENELEMENT UND HYGIENEARTIKEL
ÉLÉMENT À BOUCLE POUR ÉLÉMENT DE FIXATION DE SURFACE ET OBJET POUR HYGIÈNE

(30) Priority: 09.07.2014 JP 2014141809
(43) Date of publication of application: 17.05.2017
(73) Proprietor: 3M Innovative Properties Company, St. Paul, MN 55133-3427 (US)
(72) Inventor: MORISHITA, Kenichiro, Tokyo 141-8684 (JP); WANG, Shou-Lu Grace, Saint Paul, Minnesota 55133-3427 (US)
(74) Representative: Vossius & Partner Patentanwälte Rechtsanwälte mbB
(86) International application number: PCT/US2015/039240
(87) International publication number: WO 2016/007431

(56) References cited:
- WO-A1-92/20250
- WO-A1-95/17111
- WO-A1-2008/130807
- US-A- 5 256 231
- US-A- 5 786 060
- US-A- 5 786 060
- US-A1- 2006 019 055
- US-B1- 6 202 264
- US-B1- 6 849 142

## Description

### FIELD

The present invention relates to a surface fastener loop member and a sanitary article.

### BACKGROUND

Conventionally, surface fasteners are widely used for securing, binding, and the like of various articles such as fiber products, plastic products, paper products, industrial components, electronic components, building materials, and the like, and for example, sanitary articles (such as paper diapers or the like) with surface fasteners attached as a fastening material are known. Various engaging type surface fasteners are known, such as a surface fastener set including a male material having hook-shaped engaging elements and a female material that can engage with the engaging elements, or the like. Of these, various types of fastening materials for surface fasteners that use nonwoven materials have been conventionally proposed.

International Publication No. 2008/130807 describes a loop material for a hook and loop type fastener made of a composite nonwoven material, wherein the composite nonwoven material is a loop layer of a carded nonwoven material made of thermoplastic crimped stapled fibers, the stapled fibers are 1.5 to 6.0 dTEX, and the carded nonwoven material comprises a loop layer with a basis weight of 10 to 35 g/m², a base material layer overlaid opposite the loop layer and made of spun bonded or spun melt nonwoven material with a basis weight of 5 to 30 g/m², and a plurality of bonding regions that attach the loop layer to the base material layer and that are essentially non-breathable, and that cover 35 to 55% of the surface area of the loop material.

US Patent No. 5786060 describes a female material for a surface fastener comprising a web containing a thermal fusion bonding composite fiber body, a plurality of entangled loops formed on a first surface of the web, and a compression thermal fusion bonding layer formed on a second surface of the web, wherein the web has a fiber fineness of approximately 0.5 to 10 denier and a tensile strength of more than approximately 2 g/denier, the second surface being more dense than the first surface, and therefore, a plurality of entangled loops formed on the first surface can forcibly engage with elements formed on the surface of a male material, and the peeling strength required to separate the plurality of entangled loops from the elements formed on the surface of the male material is at least 20 gf/cm.
US Patent No. 5470424 describes a manufacturing method of a liquid impermeable breathable sheet with a fibrous surface, wherein a sheet is formed with a first fiber surface and a second fiber surface, the fibers of the first surface are fused without greatly changing the fiber of the second surface, and sufficient pressure and Z gradient temperature differential are applied to the sheet to form the fused material into a liquid impermeable nonbreathable skin, fiber is accumulated on the skin while the skin is at least semi-molten to form a fiber/skin/fiber material, and thus the liquid impermeable nonbreathable skin is changed to be breathable while remaining liquid impermeable.

### SUMMARY

There are cases where a fastening member for a surface fastener with a printing layer is used in an article with a surface fastener (for example, sanitary articles such as diapers or the like). An example of the fastening member for a surface fastener is a fastening member with a printing layer formed on one main surface of a nonwoven material, and an engaging surface that engages with a fastening member of a different surface fastener provided on another main surface, and the pattern provided on the printing layer can be seen through the nonwoven material. The main surface on the printing layer side of the fastening member for a surface fastener is fastened to the main body part of the article, and therefore, an article with a surface fastener with a pattern can be formed. However, conventionally, the printing layer provided on the nonwoven material is not necessarily clear to a satisfactory level. In addition, further cost reduction and increased flexibility of the fastening member are also desired.

It has thus been found desirable to provide a fastening member for a surface fastener, more specifically a loop member, with favorable flexibility, engaging strength between a pair of fastening members (specifically peeling strength and shear strength) (also referred to simply as engaging strength in the present disclosure), and printing properties (clear printing layer), and that can be manufactured at a low cost.

One aspect of the present invention provides a loop member for a surface fastener, containing a loop layer and a base material layer; wherein the loop layer comprises a short fiber nonwoven material; the base material layer comprises a short fiber nonwoven material, and is calendared; the ratio of the average fiber fineness of the fibers in the loop layer and the average fiber fineness of the fibers in the base material layer as expressed by [average fiber fineness of fibers in the loop layer]/[average fiber fineness of fibers in the base material layer] is 1.5 to 30, and the thickness of the base material layer is 15 µm to 100 µm.

Another aspect of the present invention provides a sanitary article containing the loop member.

Another aspect of the present invention provides a sanitary article as a diaper.

The present invention can provide a surface fastener loop member that has favorable flexibility, engaging strength, and printing properties, and can be manufactured at low cost, as well as a sanitary article and a diaper.

### BRIEF DESCRIPTION OF THE DRAWINGS

FIG. 1 is a diagram illustrating an example of the loop member according to an embodiment of the present invention; and
FIG. 2 is a diagram illustrating an example of the loop member according to an embodiment of the present invention, showing a state where the loop layer and base material layer are attached by embossing.

### DESCRIPTION

Various embodiments of the present invention are described below, but the present invention is not restricted to the following embodiments, and changes that do not depart from the scope of the patent claims are intended to be included in the present invention.

The present disclosure provides a loop member for a surface fastener, containing a loop layer and a base material layer; wherein the loop layer comprises a short fiber nonwoven material; the base material layer comprises a short fiber nonwoven material, and is calendared; the ratio of the average fiber fineness of the fibers in the loop layer and the average fiber fineness of the fibers in the base material layer as expressed by [average fiber fineness of fibers in the loop layer]/[average fiber fineness of fibers in the base material layer] is 1.5 to 30, and the thickness of the base material layer is 15 µm to 100 µm.

The loop member of the present disclosure can be used as a loop member material that forms a surface fastener by various conventionally known fastening means. In one embodiment, the surface fastener can be configured by using the loop member of the present disclosure as a female material, and then combining with a male material. In another embodiment, the surface fastener can be a pair of members where both structures, namely the male material and female material which is the loop member of the present disclosure, are present on the same surface. The loop member of the present disclosure can be directly engaged to a wall, fabric, or the like for example.

More specifically, with the loop member of the present disclosure, a short fiber nonwoven material including the loop layer functions as an engaging element, and the short fiber nonwoven material including the base material layer is intended to configure the printing surface. An example of a male material that is preferable from the perspective of enabling strong engagement with the engaging element which is the nonwoven material is a hook. The hook is configured by a protrusion protruding in the thickness direction of the surface fastener. The loop member of the present disclosure can be a portion of the fastening member configured such that the hook and the loop layer containing the short fiber nonwoven material as an engaging element that can be engaged by the hook are present on the same surface.

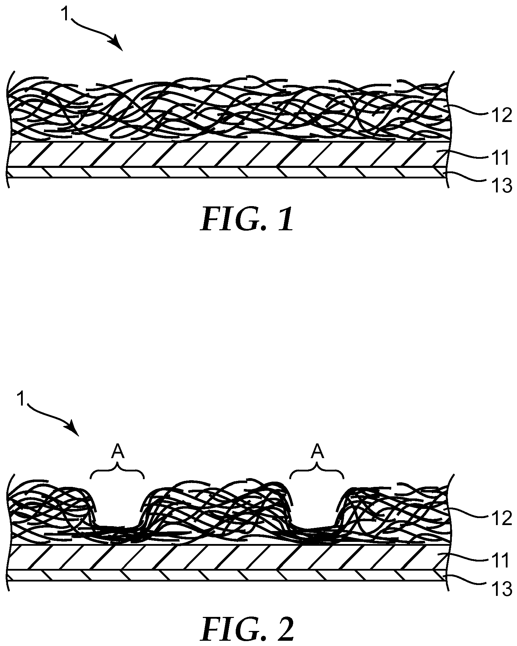

FIG. 1 is a diagram illustrating an example of the loop member according to an aspect of the present invention. With reference to FIG. 1, a loop member 1 has a base material layer 11 and a loop layer 12. In a typical embodiment, the loop member 1 can further have a printing layer 13.

The loop layer and the base material layer contain a short fiber nonwoven material. In a typical embodiment, the loop member can be configured essentially by a nonwoven material, and typically, can be configured essentially of a short fiber nonwoven material. The loop member configured essentially by the nonwoven material can have flexibility and breathability to a degree that is preferable for use as a sanitary article. The short fiber nonwoven material can form a thin and soft layer, which is advantageous. Note that in the present disclosure, the phrase "short fiber nonwoven material" is intended to be a nonwoven material where at least a major portion (over 50 mass% of the constituent fibers) are configured by staples (in other words, short fibers), and is different from nonwoven material configured by filaments (in other words, long fibers). The short fiber nonwoven material includes carded nonwoven material, air-laid nonwoven material, wet-laid nonwoven material, and the like. On the other hand, long fiber nonwoven material generally includes spun bond nonwoven material and the like. The staples are not particularly restricted, but generally can have a fiber length of several hundred mm or less.

In the loop member of the present disclosure, the loop layer and the calendared base material layer are laminated. In the present disclosure, "calendaring" is intended to be a process of adding pressure to the layer in order to smooth the layer to be processed (specifically nonwoven material which is the material of the base material layer). Therefore, calendaring in the present disclosure can included a process of passing the layer to be processed between a smoothing roller and a protruded and recessed roller (such as a thermal bond roller) to smooth the layer to be processed, or a process of passing the layer to be processed between a pair of smoothing rollers in order to smooth, and the like. Note that if the protruded and recessed roller is used for example, adjustment of the step for obtaining a desired smoothing effect (for example, performing an operation of passing the layer to be processed between the rollers a plurality of times, adjusting the point bonding conditions such that the processing surface area is relatively large in point bonding described later, and the like) can be appropriately performed by one skilled in the art.

The loop member of the present disclosure has a base material layer and loop layer that are separate layers, and therefore, the properties of each layer can be mutually controlled independently based on the purpose. The present inventors focused on the fact that in order to achieve favorable printing properties (in other words, achieving a design that is clearly printed), a highly smooth printing surface is required, but on the other hand, peeling strength and shear strength between the pair of fastening members (for example, a male member if the loop member of the present disclosure is used as the female member) must be ensured. Furthermore, the present inventors focused on the fact that the main causes that an observed printed image is unclear include: conventionally, the printing surface is not smooth because the nonwoven material is point bonded, and therefore, the ink droplets are unstable on the printing surface; and if the loop member is thick and the printing layer is observed through the loop member, the printed image appears blurred. Furthermore, the present inventors discovered that by laminating the loop layer containing the nonwoven material with high fineness as compared to the nonwoven material used in the base material layer and the base material layer that was thinned and smoothed by calendaring, the printing properties and engaging strength can be both be achieved to a high degree. A thin loop member is advantageous from the perspective of being low in cost and having excellent flexibility.

The loop layer can function as the engaging element. In one embodiment, the short fiber nonwoven material included in the loop layer can engage with the hooks. Note that examples of the hook material can include various thermoplastic resin material such as polyethylene (for example, high density polyethylene), polypropylene, polystyrene, polyvinyl chloride, polyethylene terephthalate, polybutylene terephthalate, nylon, polycarbonate, polymethyl methacrylate, polyacetal, polymethylpentene, acrylonitrile - styrene - butadiene, polyphenylene ether, polyphenylene sulfide, as well as styrene - butadiene - styrene, styrene - isoprene - styrene, and other styrene elastomers, ethylene - α-olefin copolymer and other olefin elastomers, ester elastomers, amide elastomers, urethane elastomers, vinyl chloride elastomers, silicone elastomers, fluorine elastomers, alloys thereof, and the like.

Examples of fibers configuring the short fiber nonwoven material included in the base material layer and the loop member include fibers of polyolefins (for example, polyethylene, polypropylene, and the like), polyesters (for example, PET, PBT, and the like), polyamides, polyurethanes, EVA (ethylene - vinyl - acetate), polylactic acid, rayon, copolymers and mixtures thereof, as well as natural fibers, and the like.

In one embodiment, high density polyamide is used in the loop layer from the perspective of preventing breaking of the loop layer (fiber loss and the like) due to engaging with another fastening member. On the other hand, considering material cost and environmental stability, polypropylene and polyester can also be used in the loop layer and / or base material layer.

The fibers configuring the short fiber nonwoven material can be hydrophilic or water repellent. The fibers can also be composite fibers. Examples of preferred fiber forms for the composite fibers include core-in-sheath (core-sheath (concentric and eccentric)), parallel (in other words, side-by-side), split (where the cross-section is divided into an arc shape), and the like. Furthermore, the fibers can be irregular cross-section fibers, crimped fibers, thermally shrunk fibers, and the like. The fibers can be used individually, or in combinations of two or more.

Furthermore, a two component flexible elastic composite fiber made of two components, wherein the first component is a hard elastic component made of crystalline polypropylene and the second component is a thermoplastic elastomer, or a fiber blend of the aforementioned fibers and other fibers can be suggested.

In a preferred embodiment, the fibers configuring the short fiber nonwoven material included in the loop layer and the base material layer has a core-in-sheath structure. Herein, if a short fiber nonwoven material configured from core-in-sheath composite fibers containing the core material and another material that covers the core material, fibers with a high melting point can be used as the core and fibers with a low melting point can be used as the sheath, in order to achieve composite fibers with excellent thermal adhesiveness. Examples can include fibers with a core-in-sheath structure using a polypropylene (core) and a polyethylene (sheath), fibers with a core-in-sheath structure using a polypropylene (core) and a modified polyethylene (sheath), fibers with a core-in-sheath structure using a polypropylene (core) and a modified polypropylene (sheath), and the like. In one embodiment, core-in-sheath fibers having a polypropylene core and a polyethylene sheath can be selected, from the perspective of light weight, high strength, high flexibility, and the like.

In a preferred embodiment, the average fiber fineness of the base material layer is approximately 0.5 denier or higher and approximately 3.0 denier or lower. The average fiber fineness is preferably approximately 0.5 denier or higher, approximately 0.7 denier or higher, approximately 0.9 denier or higher, or approximately 1.0 denier or higher from the perspective of maintaining mechanical strength of the base material layer and ease of manufacturing, and is preferably approximately 3.0 denier or lower, approximately 2.5 denier or lower, approximately 2.0 denier or lower, or approximately 1.5 denier or lower from the perspective of providing a smooth printing surface to contribute to favorable printing properties.

In a preferred embodiment, the average fiber fineness of the loop material layer is approximately 1.5 denier or higher and approximately 15 denier or lower. The average fiber fineness is preferably approximately 1.5 denier or higher, approximately 2.0 denier or higher, approximately 2.5 denier or higher, or approximately 3.0 denier or higher from the perspective of obtaining favorable engaging strength, and is preferably approximately 15 denier or lower, approximately 14 denier or lower, approximately 10 denier or lower, or approximately 8.0 denier or lower from the perspective of maintaining favorable flexibility of the loop member.

The ratio of the average fiber fineness of the fibers in the loop layer and the average fiber fineness of the fibers in the base material layer as expressed by [average fiber fineness of fibers in the loop layer] / [average fiber fineness of fibers in the base material layer] is approximately 1.5 or higher and approximately 30 or lower. The aforementioned ratio is approximately 1.5 or higher, preferably approximately 1.7 or higher, or approximately 2 or higher, from the perspective of compatibility between the engaging strength of the loop layer and the printing properties of the base material layer, and the aforementioned ratio is approximately 30 or lower, preferably approximately 18 or lower, or approximately 6 or lower, from the perspective of maintaining mechanical strength of the base material layer and ease of manufacturing.

The ratio is calculated based on a value measuring the average fiber fineness of the loop layer and the base material layer using a Vibromat ME (manufactured by TEXTECHNO) which is a fiber fineness measuring device, or by an equivalent measuring device. Specifically, first, approximately ten fibers are collected as randomly as possible. At this time, damaged fibers such as cut fibers and the like cannot be used in measuring, and therefore, are not collected. Of the collected fibers, an end of one fiber is pinched with tweezers, and both ends of the fiber are secured to clamps of a measuring instrument without twisting or stretching the fibers. At this time, a thread is provided to be perpendicular. At a constant fiber length and stress, a fixed oscillation rate is measured from the oscillating of the fibers secured by the clamps, and then converted to fiber fineness. The operation is repeated five times, and the average value for the fiber fineness of five fibers is used as the average fiber fineness. The measurement environment is set to a temperature of 21°C ±1°C and a humidity of 65% ±2%. The ratio as expressed by [average fiber fineness of fibers in the loop layer] / [average fiber fineness of fibers in the base material layer] is calculated based on the determined average fiber fineness of the loop layer and the base material layer.

The short fiber nonwoven material included in the base material layer and loop layer can be manufactured using a standard method as the manufacturing method of the short fiber nonwoven material, and for example, can be carded nonwoven material, air-laid nonwoven material, and the like. Herein, if long fiber nonwoven material manufactured by a spun bond method or melt blow method is used as the base material layer, a high density nonwoven material must be used in order to reduce breathability of the obtained base material layer. Furthermore, if the base material layer configured from the long fiber nonwoven material is thinned by calendaring, the obtained base material layer tends to harden as the thickness is reduced. On the other hand, if the base material layer containing the short fiber nonwoven material is thinned by calendaring, flexibility and moderate breathability can easily be provided. The bonding form of the fibers in the short fiber nonwoven material can be thermal bonding, chemical bonding, hydroentangling, needle punching, stitch bonding, steam jet, and the like. In a preferred embodiment, the base material layer is a thermal bonding nonwoven material from the perspective of being thin and having excellent flexibility.

In a typical embodiment, the loop layer and the base material layer are mutually bonded by embossing, chemical bonding, water jetting, air jets, and the like. FIG. 2 is a diagram illustrating an example of the loop member according to an aspect of the present invention, showing a state where the loop layer and base material layer are attached by embossing. As illustrated in FIG. 2, in the loop member 1, the base material layer 11 and the loop layer 12 can be mutually bonded by an embossing pattern A. Various shapes of the embossing pattern formed by embossing are possible, such as rectangles, wave shapes, and the like. For example, in the illustrative embodiment of the bonding region where the loop layer and the base material layer are bonded, if the loop layer and / or base material layer contain a carded nonwoven material, the pitch of the bonding region in the fiber direction of the carded nonwoven material is preferably provided to be shorter than the pitch of the bonding region in the direction generally orthogonal thereto. The bonding region is advantageous from the perspective that fluffing of the carded nonwoven material can be prevented. A structure is achieved where the loop layer is bonded with the calendared base material layer, and therefore, the loop layer can be stable and firmly attached to the base material layer. Therefore, when the loop member is peeled from the male member or the like, problems where the loop layer peels from the base material layer, the loop layer tears and breaks, and the like will not easily occur.

The base material layer is calendared. The calendaring method and conditions can be set based on the purpose. When the density of the base material layer increases due to calendaring, the base material layer can have a smooth surface.

The calendared base material layer is advantageous from the perspective of having a stable bond between the base material layer and the loop layer, in other words, the engaging strength of the loop member is increased by the base material layer contributing to the stable retention of the loop layer. Furthermore, the calendared base material layer can have low breathability as compared to the nonwoven material that is not calendared for example, and therefore, while a desired breathability can be maintained due to using the nonwoven material, excessively high breathability which is unfavorable in the manufacturing step can be prevented.

In a typical embodiment, the base material layer can be calendared to a degree that the layer becomes film-like (in other words, a state where smoothness of the nonwoven material surface is high, and fine printing is possible on the nonwoven material surface). The film-like base material layer after calendaring has low breathability as compared to the base material layer before calendaring.

On the other hand, in a typical embodiment, the loop layer is substantially not calendared for the purpose of surface smoothing. Thereby, the short fiber nonwoven material included in the loop layer can favorably function as the engaging element. Note that when bonding and attaching the loop layer and the base material layer for example, the loop layer being subject to compression by a roller or the like is not excluded. For example, there are cases where only one surface of the short fiber nonwoven material layer is melted and surface smoothed, and cases where the other surface of the short fiber nonwoven material layer is also simultaneously compressed and melted. In this case, the engaging strength (for example, peeling strength and shear strength) of the short fiber nonwoven material present on the other surface may be reduced. In contrast, in the loop member of the present disclosure, the loop layer is combined with the calendared base material layer, and therefore, the loop layer is not affected by the calendaring of the base material layer, and thus the loop layer can be formed without impairing the desired engaging strength.

In an embodiment of the present invention, the thickness of the base material layer is approximately 15 µm or more and approximately 100 µm or less. The thickness of the base material layer is approximately 15 µm or higher, preferably approximately 20 µm or higher, or approximately 25 µm or higher, or approximately 35 µm or higher, from the perspective of maintaining favorable mechanical strength and obtaining favorable engaging strength. The thickness is approximately 100 µm or lower, preferably approximately 85 µm or lower, approximately 70 µm or lower, or approximately 55 µm or lower, from the perspective of low cost and flexibility. In the base material layer manufactured by the short fiber nonwoven material (for example, thermal bonded nonwoven material), if the thickness is set to the aforementioned predetermined thickness by calendaring, a soft base material layer and reduction of breathability can both be achieved.

In the present disclosure, the thickness of the base material layer is measured as follows. First, a sample for thickness measurement with a surface area of 10 x 10 mm is collected from the loop member. Next, the loop layer is removed from the measurement sample, and the thickness of the base material layer that is not bonded or attached to the loop layer is measured. The thickness measurement is repeated five times at different points on the sample, and the average value of the thickness resulting from measuring five times is set as the thickness of the base material layer. For measuring the thickness, a thickness measuring instrument (Mitsutoyo ABSOKUTE (KK-547-055, or equivalent measuring instrument) is used, the sample is interposed between a cylindrical end surface and base of the measuring instrument, and then the thickness of the base material layer is measured by reading the thickness represented digitally after two seconds.

In a preferred embodiment, the basis weight of the base material layer is approximately 8 gsm or higher and approximately 30 gsm or lower. The basis weight is preferably approximately 8 gsm or higher, approximately 10 gsm or higher, approximately 12 gsm or higher, or approximately 15 gsm or higher, from the perspective of maintaining mechanical strength of the base material layer, and is preferably approximately 30 gsm or lower, approximately 25 gsm or lower, approximately 21 gsm or lower, or approximately 17 gsm or lower, from the perspective of achieving a thin and flexible low cost loop member.

In a preferred embodiment, the basis weight of the loop layer is approximately 12 gsm or higher and approximately 30 gsm or lower. The basis weight is preferably approximately 12 gsm or higher, approximately 15 gsm or higher, approximately 12 gsm or higher, or approximately 15 gsm or higher, from the perspective of achieving favorable bonding strength of the loop layer, and is preferably approximately 30 gsm or lower, approximately 25 gsm or lower, approximately 22 gsm or lower, or approximately 20 gsm or lower, from the perspective of achieving a thin and flexible low cost loop member.

The surface of a printing layer forming surface of the base material layer can be surface treated (for example, corona discharge treated or E-beam treated). Furthermore, a process such as coloring or the like can be performed on the loop layer and / or the base material layer.

The base material layer and / or loop layer can typically contain the short fiber nonwoven material, from the perspective of favorably achieving the benefits of the loop member of the present disclosure. Furthermore, additional layers other than the short fiber nonwoven material can be included. The base material layer, the loop layer, and the short fiber nonwoven material included in the base material layer or loop layer can each be a single layer or plurality of layers. Examples of additional layers include adhesive layers, resin film, knitted material, woven material, paper, laminated bodies thereof, and the like. The forming method of the additional layers is not particularly restricted, but conventionally known methods such as coating, dry laminating, extrusion laminating, wet laminating, heat laminating, ultrasonic waves, and the like can be used.

In a typical embodiment, the loop member further includes a printing layer. The printing layer can be directly attached onto the base material layer. In the present disclosure, the phrase "the printing layer is directly attached onto the base material layer" indicates that the printing layer is disposed on the base material layer without another member or layer interposed therebetween (in other words, in contact with the base material layer), and the printing layer can essentially not be peeled from the base material layer while maintaining this form. This printing layer is advantageous from the perspective of achieving a thin loop member using a simple process.

The printing layer at least has an ink layer, and is one layer or a plurality of layers. The printing layer can be configured of only the ink layer, or can have a base coat and / or top coat, in addition to the ink layer. The base coat and the top coat contribute to improving the fixing properties of the ink layer onto the base material layer.

The ink layer and the optional base coat and top coat can be present as a continuous layer on the base material layer, or can be provided discontinuously, and can be appropriately designed for a desired purpose such as a design for the purpose of the ink layer. The design can be arbitrarily selected such as a character, image, pattern, or the like.

Examples of the material of the ink layer include various conventionally known inks, and water soluble and solvent based inks are used. Resins that are normally conventionally used can be used as the resin containing the ink. Examples include acrylic resin, polyurethane resin, polyamide resin, urea resin, polyester resin, vinyl chloride resin, vinylidene chloride resin, vinyl chloride - vinyl acetate copolymer resin, ethylene - vinyl acetate copolymer resin, olefin resin, chlorinated olefin resin, epoxy resin, petroleum resin, cellulose derivative resin, and the like. If the ink layer has excellent toughness, the ink layer is less likely to slough off, even if the ink layer is exposed during use of an article having the loop member of the present disclosure. From this perspective, acrylic ink, urethane ink, and the like are preferable.

The ink layer itself preferably has adhesive properties from the perspective of fixing properties of the printing layer on the base material layer. Examples of the material for forming the ink layer having adhesive properties can include materials used in the aforementioned adhesive layer that is mixed with the ink.

The thickness of the ink layer configuring the printing layer, as well as the optional base coat and top coat are not particularly restricted, but can be approximately 0.5 µm or higher and approximately 20 µm or lower, approximately 1 µm or higher and approximately 15 µm or lower, or approximately 2 µm or higher and approximately 10 µm or lower for example, from the perspective of a tough printing layer and the feel when wearing (flexibility, breathability, and the like) when used in a sanitary article.

In one embodiment, the printing layer can be adhered to the base material layer by an adhesive. In this case, the fixing properties of the printing layer are favorable. Examples of the material of the adhesive can include acrylic polymers (for example, acrylic adhesives commercially available from Soken Chemical & Engineering), silicone polymers, rubber polymers, and other tacky polymers, and hot melt adhesives such as Jet-melt ™ EC-3748 (commercially available from Sumitomo 3M) and the like, for example. An additive such as a tackifying resin, cross-linking agent, and the like can be optionally combined with the tacky polymer.

The thickness of the adhesive can typically be approximately 5 µm to approximately 200 µm. The adhesive can be formed by drying after applying the adhesive material to the surface of the base material layer for example.

Various manufacturing methods are possible for the loop member of the present disclosure. In an illustrative embodiment, the loop member can be manufactured by a method including the steps of: preparing the short fiber nonwoven material for the loop layer and the short fiber nonwoven material for the base material layer; calendaring the short fiber nonwoven material for the base material layer; laminating the calendared short fiber nonwoven material and the short fiber nonwoven material for the loop layer, and then mutually bonding the material to obtain a laminated web having the base material layer and the loop layer; and optionally forming the printing layer on the base material layer side of the laminated web.

Manufacturing of the short fiber nonwoven material can be performed by forming a layer by carding and then forming a web by thermal fusion bonding using air jets or thermal bonding.

Calendaring is performed under conditions set such that the short fiber nonwoven material for the base material layer is compressed to a desired thickness. In the illustrative embodiment, if the base material layer is configured by polypropylene / polyethylene core-in-sheath fibers, calendaring can be performed under roller temperature conditions of approximately 100°C to approximately 180°C.

Next, the short fiber nonwoven material of the calendaring agent and the short fiber nonwoven material for the loop layer are laminated, and then mutually bonded by partial thermal compression bonding or the like to obtain a laminated web for example. For the bonding conditions, a temperature within a range of approximately 110°C to approximately 180°C, and a pressure with a range of 0 N/m² to approximately 1000 N/m² are possible.

An example of the method of forming the printing layer on the base material layer includes a method of directly coating the material for forming the printing layer (referred to as printing material in the present disclosure) onto the base material layer. Furthermore, a method of transferring the printing layer formed on a liner in advance onto the base material layer, and then removing the liner, and the like can be used. The method based on transferring is preferable from the perspective of the ink in the printing layer being less likely to penetrate the base material layer and the loop layer.

Specifically for the method based on transferring, first, the printing layer is formed on a liner. The liner preferably has a surface with moderate release properties in order to transfer the printing layer to the base material layer. Various sheets which are conventionally known as liners for transferring can be used, and examples include silicone coated craft paper, silicone coated polyethylene coated paper, silicone coated or non-coated polymer material (for example, polyethylene, polypropylene, and the like), polymer peeling agents such as base materials coated with silicone urea, urethanes, and long-chain alkyl acrylates, and the like. Suitable commercially available peeling liners include liners sold under the product name "Polysilk" from Rexam Release of Oakbrook, Illinois, liners sold under the product name "Exhere" from P.H. Glatfelter Company of Spring Grove, Pennsylvania, and the like. The liner can be embossed or the like on the printing layer forming surface for example.

The printing material is applied on the liner. In a typical embodiment, the ink is applied on the liner, using an arbitrary printing method such as roll coating, gravure coating, curtain coating, spray coating, screen printing, and the like. Alternatively, the top coat, ink, and base coat can be applied in this order on the liner for example. The sheet for transferring made by forming the printing layer on the liner is obtained according to the aforementioned procedure.

Next, in order for the printing layer side of the sheet for transferring and the base material layer side of the laminated web to correspond, the sheet for transferring and the laminated web pass between a pair of rollers, and therefore, the printing layer is transferred from the liner to the base material layer. Thereby, a loop member where the printing layer is formed on the base material layer can be obtained.

Note that if the printing layer has an ink layer and base coat and / or top coat, the printing layer can be sequentially formed by a plurality of steps. For example, if the printing layer contains the base coat, ink layer, and top coat, the base coat can be formed on the base material layer in advance, then the ink layer can be transferred, and then the top coat can be formed. In the aforementioned method, the printing layer can be directly attached to the base material layer.

In a preferred embodiment, the basis weight of the loop member is approximately 20 gsm or higher and approximately 60 gsm or lower. The basis weight is preferably approximately 12 gsm or higher, approximately 15 gsm or higher, approximately 12 gsm or higher, or approximately 15 gsm or higher, from the perspective of achieving favorable bonding strength of the loop member, and is preferably approximately 60 gsm or lower, approximately 50 gsm or lower, approximately 43 gsm or lower, or approximately 37 gsm or lower, from the perspective of achieving a thin and flexible low cost loop member.

In a preferred embodiment, the peeling strength between the loop member and another fastening member fastened thereto is approximately 1 N / 25.4 mm or higher and approximately 10 N / 25.4 mm or lower. The peeling strength is the 90° peeling strength measured in accordance with JTM-1221, using a 1600 pins / square inch hook member (manufactured by 3M Company, 1600DH) as the male material. The peeling strength is preferably approximately 1 N / 25.4 mm or higher, approximately 1.5 N / 25.4 mm or higher, approximately 2.5 N / 25.4 mm or higher, or approximately 3 N / 25.4 mm or higher from the perspective of favorable engaging strength, and is preferably approximately 10 N / 25.4 mm or lower, approximately 8 N / 25.4 mm or lower, approximately 7 N / 25.4 mm or lower, or approximately 6 N / 25.4 mm or lower from the perspective of a favorable feel during use.

In a preferred embodiment, the shear strength between the loop member and another fastening member fastened thereto is approximately 25 N / 20 mm X 25.4 mm or higher. The shear strength is the value measured in accordance with JTM-1235, using a 1600 pins / square inch hook member (manufactured by 3M Company, 1600DH) as the male material. The shear strength is preferably approximately 25 N / 20 mm X 25.4 mm or higher, approximately 30 N / 20 mm X 25.4 mm or higher, approximately 35 N / 20 mm X 25.4 mm or higher, or approximately 40 N / 20 mm X 25.4 mm from the perspective of favorable engaging strength. The upper limit of the shear strength is not particularly restricted, but from the perspective of ease of manufacturing, is preferably approximately 80 N / 20 mm X 25.4 mm or lower.

In a preferred embodiment, the tensile strength of the loop member is controlled to a predetermined range based on the purpose. If the tensile strength of the loop member is too high, the loop member tends to harden. On the other hand, if the tensile strength of the loop member is too low, there is concern that problems may occur where the loop member stretches in the MD direction during manufacturing of the sanitary article (diaper or the like) (if the tensile strength is too low in the MD direction), or where the loop member stretches due to shearing of the hooks combined with the loop member during use of a sanitary article (diaper or the like) having the loop member (if the tensile strength is too low in the CD direction). Note that in the present disclosure, "MD direction of the loop member" refers to the machine direction during manufacturing of the loop member. The MD direction during manufacturing of the loop member normally matches the MD direction during manufacturing of the loop layer and the base material layer. Furthermore, "CD direction" refers to the direction orthogonal to the MD direction (in other words, at 90°). From the aforementioned perspective, the tensile strength is preferably approximately 7 N / 25.4 mm or higher, approximately 10 N / 25.4 mm or higher, or approximately 12 N / 25.4 mm or higher, and preferably approximately 30 N / 25.4 mm or lower, approximately 25 N / 25.4 mm or lower, or approximately 20 N / 25.4 mm or lower, in the MD direction. Furthermore, the tensile strength is preferably approximately 2.5 N / 25.4 mm or higher, approximately 3 N / 25.4 mm or higher, or approximately 3.5 N / 25.4 mm or higher, and preferably approximately 15 N / 25.4 mm or lower, approximately 25 N / 25.4 mm or lower, or approximately 10 N / 25.4 mm or lower, in the CD direction. Note that the tensile strength is the tensile strength during 5% elongation, measured by a method described in the "Examples" section of the present disclosure or a method known by one skilled in the art to be equivalent.

The loop member of the present disclosure can be used in securing various articles such as various articles such as flooring, wall material, clothing, cleaning members, automobile interior materials, and the like. The loop member of the present disclosure can have favorable flexibility and breathability due to the configuration thereof, and therefore, is particularly suitable as a loop member for sanitary articles such as paper diapers, sanitary napkins, breast pads, and the like.

Another embodiment of the present invention provides a sanitary article containing a surface fastener that contains the loop member according to one of the aforementioned embodiments of the present invention.

Examples of the sanitary article include children and adult diapers, sanitary napkins, and napkins for other applications, and the like. In a typical embodiment, the sanitary article is a diaper. In a typical embodiment, the loop member of the present disclosure can be used as the surface fastener in a sanitary article, preferably a diaper, by combining with a loop member of the present disclosure or another arbitrary fastening member.

In a preferred embodiment, the sanitary article has excellent breathability. More specifically, the breathability of the sanitary article as measured by the Gurley method is preferably approximately 5 seconds or less, from the perspective of being able to provide a favorable feel when wearing the sanitary article. The breathability is more preferably approximately 3 seconds or less, and even more preferably approximately 1 second or less. The lower limit is not particularly restricted, but in some embodiments, is approximately 0.1 seconds or more.

The manufacturing method of the sanitary article is not particularly restricted, but the following method can be used. Arbitrary conventionally known components can be used as components other than the loop member in the sanitary article, and the details are not described herein. In the sanitary article, a method of attaching the loop member to a part to be applied can be an arbitrary conventionally known method. The printing layer forming surface of the loop member is fastened to the part to be applied by a conventionally known fastening method (adhesion by glue, thermal fusion bonding, ultrasonic processing, and the like, and mechanical attachment by sewing, staples, and the like). When attaching by glue, a known adhesive such as SIS, SBS, or other rubber, acrylic, silicone, EVA, or the like is appropriately selected as needed, but there is no restriction to just these resins.

### EXAMPLES

Hereinafter, the present invention is further described using examples, but the present invention is not limited to these examples.

### <Preparing the loop member>

### Example 1

### (Nonwoven material)

Loop layer: Carded nonwoven material (product number: ESC236CSDL, average fiber fineness 5.4 denier, 51 mm) configured with polyethylene (sheath) / polypropylene (core) biconstituent fiber, commercially available from FiberVisions Corporation. (Georgia, USA)

Base material layer: Carded nonwoven material (product number: ESC215CA, average fiber fineness 1.5 denier, 51 mm) configured with polyethylene (sheath) / polypropylene (core) biconstituent fiber, commercially available from FiberVisions Corporation.

### (Calendaring process)

The calendaring process for producing the base material layer was performed by feeding the aforementioned carded nonwoven material prepared for the base material layer in between the metal smoothing roller and the cotton roller and heating the metal rollers to the temperature shown below.
Metal smoothing roller temperature: 315°F
Cotton roller temperature: Room temperature
Cotton roller (Refiller by Holyoke)
Surface hardness: 85 (durometer, type D)
Material: 50% cotton, 50% linen

### (Preparing laminated web)

The calendaring processed carded nonwoven material that was obtained and the carded nonwoven material for loop layer were overlaid and fed in between a pattern roller with a waveform pattern and a metal smoothing roller, and thermal compression bonded under the conditions where the pattern roller temperature was 290°F and the metal smoothing roller temperature was 300°F, to obtain a laminated web. The surface area ratio of the waveform pattern part was 30% (therefore, 30% of the surface area was thermal compression bonded).

### (Printing)

The printing layer was formed by flexographic printing on the base material layer side of the laminated web.

The following procedure was performed to obtain the loop member.

### Example 2

For the base material layer, 1 cycle of a process that point bonds an area that is 35% of the surface was performed instead of the calendaring process. The temperature of the point bonding roller and the smoothing roller was 300°F. All other procedures for preparing the loop members were the same as Example 1.

### Example 3

For the base material layer, 2 cycles of a process that point bonds an area that is 35% of the surface was performed instead of the calendaring process. The temperature of the point bonding roller and the smoothing roller was 300°F. All other procedures for preparing the loop members were the same as Example 1.

### Reference Example 1

A nonwoven material (commercially available, base material layer not calendar processed) with a basis weight of 47 gsm was used, wherein the loop layer is a short fiber nonwoven material with an average fiber fineness of 6 denier that is a core-in-sheath type with polyethylene (sheath) / polypropylene (core) biconstituent fiber, and the base material layer is a short fiber nonwoven material with an average fiber fineness of 1.5 denier that is a core-in-sheath type with polypropylene (sheath) / polypropylene (core) biconstituent fiber.

### Reference Example 2

A nonwoven material (commercially available, base material layer not calendar processed) with a basis weight of 43 gsm was used, wherein the loop layer is a short fiber nonwoven material with an average fiber fineness of 6 denier that is a core-in-sheath type with polyethylene (sheath) / polypropylene (core) biconstituent fiber, and the base material layer is a short fiber nonwoven material with an average fiber fineness of 1.5 denier that is a core-in-sheath type with polypropylene (sheath) / polypropylene (core) biconstituent fiber.

### Reference Example 3

A nonwoven material with a basis weight of 45 gsm was collected from a diaper (commercially available). Here, the collected nonwoven material was prepared by placing the carded polypropylene fiber with an average fiber fineness of 4 denier onto the polypropylene nonwoven material formed by the melting process which is the base material layer, and emboss processed (base material layer not calendaring processed).

### <Property evaluation>

### 1. 90° peeling strength

The 90° peeling strength was the value measured in accordance with JTM-1221, using a 1600 pins / square inch hook member (manufactured by 3M Company, 1600DH) as the male material.

### 2. Sheer strength

The shear strength was the value measured in accordance with JTM-1235, using a 1600 pins / square inch hook member (manufactured by 3M Company, 1600DH) as the male material.

### 3. Tensile strength

Tensile strength in the MD direction and the CD direction of the tape member was measured using a Tensilon universal testing machine (RTG-1225 produced by AND (A&D)) with the method shown below.

The tape members were cut into the lengths below by a 25 mm wide leather cutter to prepare samples.
MD direction tensile strength measurement: 35 mm or more in length, 25.4 mm in width
CD direction tensile strength measurement: 120 mm or more in length, 25.4 mm in width The settings for the Tensilon were as follows.
Chuck interval: 25 mm (for MD direction tensile strength measurements), 100 mm (for CD direction tensile strength measurements)
Tensile speed: 300 mm / minute

A reinforcing tape (25 mm width filament tape (manufactured by 3M Company, Scotch (registered trademark) 898)) was pasted to the printing layer side of a chuck gripping part of the sample, a 25 mm filament tape (manufactured by 3M Company, Scotch (registered trademark) 898)) was similarly pasted to the loop layer side, and then the sample was attached to the chuck.

The tensile strength during 5% elongation was recorded as the tensile strength value.

### 4. Visibility

The printing layer was visually observed from the loop layer surface, and then evaluated by the following criteria.
Excellent: The detailed design portion of the printing layer is all clear
Good: A part of the detailed design portion of the printing layer is clear
Acceptable: The design of the printing layer can be confirmed, but the detailed design portion is not clear

The results are shown in Table 1.

**Table 1**

| | | Example 1 | Example 2 | Example 3 | Reference Example 1 | Reference Example 2 | Reference Example 3 |
|---|---|---|---|---|---|---|---|
| Average Fiber Fineness (Denier) Loop Layer / Base Material Layer | | 5.4 / 1.5 | 5.4 / 1.5 | 5.4 / 1.5 | 6/1.5 | 6/1.5 | 4 |
| Loop Layer / Base Material Layer Average Fiber Fineness Ratio | | 4 | 4 | 4 | 4 | 4 | - |
| Thickness | Base Material Layer | 40 | 83 | 77 | 101 | 101 | - |
| Basis Weight | Base Material layer | 20 | 20 | 20 | 26 | 22 | - |
| | Loop Layer | 17 | 17 | 17 | 21 | 21 | - |
| | Total Basis Weight | 37 | 37 | 37 | 47 | 43 | 45 |
| 90° Peeling Strength (N / 25.4 mm) | | 4 | 3.2 | 3.5 | 3.5 | 3.5 | 2 |
| Shear Strength (N / 20 mm X 25.4 mm) | | 48 | 40 | 42 | 50 | 40 | 39 |
| Tensile Strength (N / 25.4 mm) | | MD: 15 CD: 6 | - | - | - | - | - |
| Visibility | | Excellent | Good | Good | Yes | Yes | Yes |

The loop member of the present disclosure can be suitably applied, for example, to sanitary articles such as children and adult diapers, sanitary napkins, napkins for other applications, and the like.

## Claims

1. A loop member for a surface fastener, comprising a loop layer and a base material layer; wherein
the loop layer comprises a short fiber nonwoven material;
the base material layer comprises a short fiber nonwoven material, and is calendared;
the ratio of the average fiber fineness of the fibers in the loop layer and the average fiber fineness of the fibers in the base material layer as expressed by [average fiber fineness of fibers in the loop layer]/[average fiber fineness of fibers in the base material layer] is 1.5 to 30, and the thickness of the base material layer is 15 µm to 100 µm.

2. The loop member according to claim 1, wherein the loop layer and the base material layer are mutually attached by embossing.

3. The loop member according to claim 1 or 2, wherein the base material layer is a thermal bonded nonwoven material.

4. The loop member according to any one of claims 1 through 3, wherein the basis weight is 20 gsm or higher and 60 gsm or lower.

5. The loop member according to any one of claims 1 through 4, wherein
the base material layer has a basis weight of 8 gsm or higher and 30 gsm or lower; and
the loop layer has a basis weight of 12 gsm or higher and 30 gsm or lower.

6. The loop member according to any one of claims 1 through 5, wherein the base material layer and the loop layer both contain core-in-sheath fibers with a polypropylene core and a polyethylene sheath.

7. The loop member according to any one of claims 1 through 6, further comprising a printing layer provided on the base material layer.

8. A sanitary article, comprising a loop member according to any one of claims 1 through 7.

9. The sanitary article according to claim 8, which is a diaper.

## Patentansprüche

1. Schlaufenelement für ein Oberflächenbefestigungselement, das eine Schlaufenschicht und eine Basismaterialschicht aufweist; wobei
die Schlaufenschicht ein Kurzfaservliesmaterial aufweist;
die Basismaterialschicht ein Kurzfaservliesmaterial aufweist und kalandriert ist;
das Verhältnis der durchschnittlichen Faserfeinheit der Fasern in der Schlaufenschicht zu der durchschnittlichen Faserfeinheit der Fasern in der Basismaterialschicht, ausgedrückt durch [durchschnittliche Faserfeinheit der Fasern in der Schlaufenschicht ]/[durchschnittliche Faserfeinheit der Fasern in der Basismaterialschicht] 1,5 bis 30 beträgt, und die Dicke der Basismaterialschicht 15 µm bis 100 µm beträgt.

2. Schlaufenelement nach Anspruch 1, wobei die Schlaufenschicht und die Basismaterialschicht durch Prägen aneinander befestigt sind.

3. Schlaufenelement nach Anspruch 1 oder 2, wobei die Basismaterialschicht ein thermisch gebundenes Vliesmaterial ist.

4. Schlaufenelement nach einem der Ansprüche 1 bis 3, wobei das Flächengewicht 20 g/m² oder mehr und 60 g/m² oder weniger beträgt.

5. Schlaufenelement nach einem der Ansprüche 1 bis 4, wobei
die Basismaterialschicht ein Flächengewicht von 8 g/m² oder mehr und 30 g/m² oder weniger aufweist; und
die Schlaufenschicht ein Flächengewicht von 12 g/m² oder mehr und 30 g/m² oder weniger aufweist.

6. Schlaufenelement nach einem der Ansprüche 1 bis 5, wobei sowohl die Basismaterialschicht als auch die Schlaufenschicht Kern-in-Mantel-Fasern mit einem Polypropylenkern und einer Polyethylenhülle enthalten.

7. Schlaufenelement nach einem der Ansprüche 1 bis 6, das ferner eine Druckschicht aufweist, die auf der Basismaterialschicht vorgesehen ist.

8. Hygieneartikel, der ein Schlaufenelement nach einem der Ansprüche 1 bis 7 aufweist.

9. Hygieneartikel nach Anspruch 8, bei dem es sich um eine Windel handelt.

## Revendications

1. Élément à boucles pour un élément de fixation de surface, comprenant une couche de boucles et une couche de matériau de base ; dans lequel
la couche de boucles comprend un matériau non tissé à fibres courtes ;
la couche de matériau de base comprend un matériau non tissé à fibres courtes, et est calandrée ;
le rapport de la finesse moyenne de fibre des fibres dans la couche de boucles et la finesse moyenne de fibre des fibres dans la couche de matériau de base tel qu'exprimé par [finesse moyenne de fibre des fibres dans la couche de boucles]/[finesse moyenne de fibre des fibres dans la couche de matériau de base] va de 1,5 à 30, et l'épaisseur de la couche de matériau de base va de 15 µm à 100 µm.

2. Élément à boucles selon la revendication 1, dans lequel la couche de boucles et la couche de matériau de base sont mutuellement fixées par gaufrage.

3. Élément à boucles selon la revendication 1 ou 2, dans lequel la couche de matériau de base est un matériau non tissé à liaison thermique.

4. Élément à boucles selon l'une quelconque des revendications 1 à 3, dans lequel la masse surfacique est de 20 g/m² ou plus élevée et de 60 g/m² ou plus basse.

5. Élément à boucles selon l'une quelconque des revendications 1 à 4, dans lequel
la couche de matériau de base a une masse surfacique de 8 g/m² ou plus élevée et 30 g/m² ou plus basse ; et
la couche de boucles a une masse surfacique de 12 g/m² ou plus élevée et 30 g/m² ou plus basse.

6. Élément à boucles selon l'une quelconque des revendications 1 à 5, dans lequel la couche de matériau de base et la couche de boucles contiennent l'une et l'autre des fibres âme-dans-gaine avec une âme en polypropylène et une gaine en polyéthylène.

7. Élément à boucles selon l'une quelconque des revendications 1 à 6, comprenant en outre une couche d'impression fournie sur la couche de matériau de base.

8. Article hygiénique, comprenant un élément à boucles selon l'une quelconque des revendications 1 à 7.

9. Article hygiénique selon la revendication 8, qui est une couche.
